**Europäisches Patentamt**

**European Patent Office**

**Office européen des brevets**

(19)

(11) Veröffentlichungsnummer : **0 082 935**
**B1**

(12) **EUROPÄISCHE PATENTSCHRIFT**

(45) Veröffentlichungstag der Patentschrift :
26.09.84

(51) Int. Cl.³ : **C 07 C 47/02,** C 07 C 45/62,
B 01 J 23/44

(21) Anmeldenummer : 82109760.7

(22) Anmeldetag : 22.10.82

(54) Verfahren zur kontinuierlichen Herstellung von n-Butyraldehyd durch selektive Hydrierung von Crotonaldehyd in flüssiger Phase in Gegenwart eines Palladium-Aluminiumoxid-Katalysators.

(30) Priorität : 23.12.81 DE 3151086

(43) Veröffentlichungstag der Anmeldung :
06.07.83 Patentblatt 83/27

(45) Bekanntmachung des Hinweises auf die Patenterteilung : 26.09.84 Patentblatt 84/39

(84) Benannte Vertragsstaaten :
BE DE FR GB IT NL

(56) Entgegenhaltungen :
CH-A-  467 229
FR-A- 1 172 835

(73) Patentinhaber : CHEMISCHE WERKE HÜLS AG
- RSP Patente / PB 15 - Postfach 13 20
D-4370 Marl 1 (DE)

(72) Erfinder : Fischer, Lothar, Dr.
Leverkusener Strasse 15
D-4370 Marl 1 (DE)
Erfinder : zur Hausen, Manfred, Dr.
Höchster Strasse 1
D-4370 Marl 1 (DE)
Erfinder : Wember, Kurt, Dr.
Griesheimer Strasse 14
D-4370 Marl 1 (DE)

Jouve, 18, rue St-Denis, 75001 Paris, France

**Beschreibung**

Bei der selektiven katalytischen Hydrierung von Crotonaldehyd mit Hilfe von Platinmetall-Katalysatoren zu n-Butyraldehyd entstehen durch Bildung von Kohlenmonoxid und Propylen Verluste von etwa 5 %. Diese Nachteile vermeidet man nach dem Verfahren der DE-AS 10 70 160 (= US-PSS 2 930 765 und 2 930 766) durch den Einsatz von Katalysatoren der Platinmetalle auf leicht alkalisch wirkenden Trägern. Die selektive Hydrierung von Crotonaldehyd zu n-Butyraldehyd erfolgt bei diesem Verfahren in der Gasphase bei Normaldruck. Man erreicht jedoch nur unbefriedigende Raum-Zeit-Ausbeuten.

Es besteht daher ein großes Interesse an einem verbesserten Verfahren zur selektiven Hydrierung von Crotonaldehyd mit höheren Raum-Zeit-Ausbeuten. Die sich daraus ergebende Aufgabe wird erfindungsgemäß entsprechend den Angaben der Ansprüche gelöst.

Überraschenderweise erhält man durch Hydrierung von Crotonaldehyd in flüssiger Phase in Gegenwart eines in besonderer Weise hergestellten Palladium-Aluminiumoxid-Träger-Katalysators selektiv n-Butyraldehyd in hoher Raum-Zeit-Ausbeute bei nahezu quantitativem Umsatz des Crotonaldehyds. Die Bildung von gasförmigen Nebenprodukten konnte nicht festgestellt werden.

Der erfindungsgemäß erhaltene n-Butyraldehyd ist überraschenderweise von so hoher Reinheit bzw. frei von störenden Nebenprodukten, daß man ihn ohne destillative Reinigung auf übliche Weise kontinuierlich in 2-Ethylhexenal überführen kann, das man anschließend in bekannter Weise zum 2-Ethylhexanol-1 hydrieren kann. Die Ausbeute an 2-Ethylhexanol beträgt 96 % und liegt damit ebenso hoch wie beim Einsatz von reinem n-Butyraldehyd aus der Oxo-Synthese.

Obwohl man bei dem erfindungsgemäßen Verfahren in flüssiger Phase die Verdampfung des Crotonaldehyds, die eine Reinigung bewirkt, einspart, ist selbst nach langem Katalysatoreinsatz, beispielsweise nach 8 Monaten, kein Nachlassen der Kontaktaktivität festzustellen.

Der Crotonaldehyd wird in flüssiger Phase bei einem absoluten Druck von 5 bis 50 bar, vorzugsweise 10 bis 20 bar, und einer Temperatur von 20 bis 100 °C, vorzugsweise 40 bis 60 °C, an einem Palladium-Aluminiumoxid-Träger-Katalysator selektiv hydriert. Das Palladium ist in einer Randzone des Aluminiumoxids, das als Träger fungiert, abgeschieden. Als Aluminiumoxid wird vorzugsweise die $\gamma$-Modifikation verwendet. Bei dem für das erfindungsgemäße Verfahren geeigneten Aluminiumoxid entfallen 40 bis 60 %, vorzugsweise 45 bis 55 %, insbesondere 50 % des spezifischen Porenvolumens auf Poren, deren Durchmesser größer als 7,5 nm ist bei einem gesamten spezifischen Porenvolumen von 0,4 bis 0,6, vorzugsweise 0,45 bis 0,50 cm$^3$/g und einer spezifischen inneren Oberfläche von 130 bis 160, vorzugsweise 140 bis 155 m$^2$/g, insbesondere 150 m$^2$/g. Der Palladiumgehalt liegt bei 0,1 bis 0,6, vorzugsweise bei 0,3 bis 0,5 Gewichtsprozent bei einer Dicke der Randzone von 0,05 bis 0,2 mm, wie sie für die erfindungsgemäße Reaktion erforderlich ist.

Die Form des Katalysators ist ohne Einfluß auf das katalytische Verhalten. Die Größe der Partikel spielt jedoch eine entscheidende Rolle, wenn der Umsatz auf das Katalysatorbettvolumen bezogen wird. Mit zunehmender Größe der Partikel sinkt der Umsatz, so daß man im allgemeinen bestrebt ist, die Partikel möglichst klein zu wählen. Dieses wiederum erhöht den Druckverlust, weshalb ein Kompromiß zwischen Umsatz und Druckverlust geschlossen werden muß. Die Größe der Katalysator-Partikel beträgt 1 bis 5 mm, bevorzugt 2 bis 4 mm.

Bei dem erfindungsgemäßen Verfahren erreicht man Raum-Zeit-Ausbeuten von 0,8 bis 0,9 l Aldehyl/l Kontakt · h sowie Ausbeuten an n-Butyraldehyd von 98 % bis 99 %, bezogen auf Crotonaldehyd, bei praktisch 100 %igem Umsatz. Als Nebenprodukt fällt 2-Ethylhexenal an, das bei der anschließenden Umsetzung zu 2-Ethylhexenol nicht stört. Gasförmige Nebenprodukte wurden nicht festgestellt.

Beispiel 1a

1,25 kg $\gamma$-Aluminiumoxid in Strangform (Durchmesser : 3,2 mm) mit einem spez. Porenvolumen von 0,45 cm$^3$/g, davon entfällt 45 % auf Poren, deren Durchmesser größer als 7,5 nm ist, und einer spez. inneren Oberfläche von 153 m$^2$/g werden in einer Dragéetrommel bei 90 bis 100 °C mit 800 cm$^3$ einer wäßrigen Lösung, die 6,3 g Palladium in Form von Palladium(II) chlorid und 1,1 g Chlorwasserstoff enthält, unter Drehen besprüht. Die Masse wird 15 min nachgetrocknet und bei 350 °C im Luftstrom calciniert. Man erhält 1,26 kg eines Palladium-Aluminiumoxid-Katalysators, der 0,5 Gewichtsprozent Palladium in einer Randzone des Trägerkorns von 0,11 ± 0,04 mm Dicke enthält und dessen Partikel einen Durchmesser von 3,2 mm haben.

Beispiel 1b

4,07 kg Aluminiumoxid in Strangform (Durchmesser : 1,6 mm) mit einem spez. Porenvolumen von 0,44 cm$^3$/g, davon entfällt 50 % auf Poren, deren Durchmesser größer als 7,5 nm ist, und einer spez. inneren Oberfläche von 145 m$^2$/g werden in einer Dragéetrommel bei 95 bis 105 °C mit 2,6 l einer wäßrigen Lösung, die 20,5 g Palladium in Form von Palladium(II)nitrat und 60 g HNO$_3$ enthält, unter Drehen besprüht. Die Masse wird 16 h bei 110 °C nachgetrocknet und schließlich 4 h bei 450 °C im Luftstrom calciniert. Man erhält 3,91 kg eines Palladium-Aluminiumoxid-Katalysators, der 0,5 Gewichtsprozent Palladium in einer Randzone des Trägerkorns von 0,10 ± 0,05 mm Dicke enthält und dessen Partikel einen Durchmesser von 1,6 mm haben.

Beispiel 1c

200 cm³ des nach den Angaben des Beispiels 1 a hergestellten Katalysators mit 0,5 % Palladium behandelt man zunächst bei 200 °C mit Wasserstoff, um die abgeschiedene Palladiumverbindung zu Palladium zu reduzieren. Danach wird der Katalysator in einen Reaktor gefüllt, wobei er mit Inertmaterial unter- und überschichtet wird. Der Reaktor besteht aus einem ummantelten Rohr mit einem inneren Durchmesser von 24 mm und einer Länge von 2,60 m. Im Mantel zirkuliert eine Wärmeträgerflüssigkeit. Nun werden flüssiger Crotonaldehyd (Wassergehalt : 9 %) und Wasserstoff von oben nach unten über den Katalysator geleitet, wobei der Durchsatz an flüssigem Crotonaldehyd 150 g/h und der an gasförmigem Wasserstoff, unter N. B. gerechnet, 48 l/h beträgt. Der absolute Druck liegt bei 16 bar und die Temperatur bei 50 °C. Man erhält einen Produktstrom von 153 g/h. Er besteht zu 94,3 Gewichtsprozent aus einer organischen Phase mit einem Wassergehalt von 3 Gewichtsprozent und einer Säurezahl von 0,4 mg Kaliumhydroxid/g und zu 5,7 Gewichtsprozent aus einer wäßrigen Phase, deren Wassergehalt 95 Gewichtsprozent beträgt. Die wasserfreie organische Phase besteht zu 98,5 Gewichtsprozent aus n-Butyralhdehyd, 0,4 Gewichtsprozent Butanol, 0,1 Gewichtsprozent Crotonaldehyd und 1,0 Gewichtsprozent Nebenprodukten, im wesentlichen 2-Ethylhexenal.

Während einer Versuchsdauer von 5 Monaten wurde keine Änderung der vorstehenden Zusammensetzung des Produktstroms beobachtet.

Umsatz : 100 %
Ausbeute : 98,3 %
Raum-Zeit-Ausbeute :    0,84 l Butyraldehyd/l Katalysator/h

Setzt man den nach den Angaben des Beispiels 1b erhaltenen Katalysator ein, erhält man vergleichbare Ergebnisse.

Beispiel 2a

1,25 kg γ-Aluminiumoxid in Form von Strängen (Durchmesser : 1,6 mm) mit einem spez. Porenvolumen von 0,58 cm³/g, wovon 48 % auf Poren entfallen, deren Durchmesser > 7,5 nm ist, und mit einer spez. inneren Oberfläche von 160 m²/g besprüht man in einer Dragéetrommel unter Drehen bei 90 bis 100 °C mit 800 cm³ einer wäßrigen Lösung, die 1,25 g Palladium in Form von Palladium(II) nitrat und 18 g HNO₃ enthält. Die Masse trocknet man 16 h bei 110 °C nach und calciniert abschließend 4 h bei 450 °C im Luftstrom. Man erhält 1,25 kg eines Palladium-Aluminiumoxid-Katalysators, der 0,1 Gewichtsprozent Palladium in einer Randzone des Trägerkorns von 0,1 mm Dicke enthält.

Beispiel 2b

Nach den Angaben des Beispiels 1c führt man die Hydrierung von Crotonaldehyd mit 9 % Wasser mit Hilfe von 200 cm³ des nach den Angaben des Beispiels 2a hergestellten γ-Aluminiumoxid-Träger-Katalysators, der 0,1 % Palladium enthält, bei 90 °C mit einem Wasserstoffdruck von 40 bar selektiv zum n-Butyraldehyd durch.

Die übrigen Maßnahmen entsprechen den Angaben des Beispiels 1c. Über eine Versuchsdauer von 2 Monaten ist der Umsatz praktisch vollständig. Die gaschromatographisch bestimmte Ausbeute an n-Butyraldehyd beträgt 98,6 %.

Beispiel 3a

1,25 kg γ-Aluminiumoxid in Strangform (Durchmesser : 3,2 mm) mit einem spez. Porenvolumen von 0,50 cm³/g (44 % der Poren entfallen auf solche mit einem Durchmesser von > 7,5 nm) und einer spez. inneren Oberfläche von 140 m²/g besprüht man in einer Dragéetrommel unter Drehen bei 90 bis 100 °C mit 800 cm³ einer wäßrigen Lösung, die 7,5 g Palladium in Form von Palladium(II)nitrat und 18 g HNO₃ enthält. Die Masse wird — wie unter 2a beschrieben — getrocknet und calciniert. Man erhält 1,26 kg eines Palladium-Aluminiumoxid-Katalysators, der 0,6 Gewichtsprozent Palladium in einer Randzone des Trägerkorns von 0,15 mm Dicke enthält.

Beispiel 3b

Nach den Angaben des Beispiels 1c hydriert man Crotonaldehyd mit 9 % Wasser mit Hilfe von 200 cm³ des nach den Angaben des Beispiels 3a hergestellten γ-Aluminiumoxid-Trägers, der 0,6 % Palladium enthält, bei 30 °C und einem Wasserstoffdruck von 5 bas 8 Monate lang selektiv zum n-Butyraldehyd. Die übrigen Maßnahmen entsprechen den Angaben des Beispiels 1c. Bei einem praktisch vollständigen Umsatz während der gesamten Versuchsdauer liegt die n-Butyraldehyd-Ausbeute bei 98,1 bis 98,6 %.

**Ansprüche**

1. Verfahren zur kontinuierlichen Herstellung von n-Butyraldehyd durch selektive Hydrierung von Crotonaldehyd, dadurch gekennzeichnet, daß man die Hydrierung in flüssiger Phase in Gegenwart eines Palladium-Aluminiumoxid-Träger-Katalysators, der das Palladium in einer Randzone des Trägerkorns von 0,05 bis 0,2 mm Dicke enthält, dessen Träger bei einem gesamten spez. Porenvolumen von 0,4 bis 0,6 cm³/g, 40 bis 60 % des Porenvolumens in Poren mit einem Durchmesser über 7,5 nm bei einer spez. inneren Oberfläche von 130 bis 160 m²/g besitzt und dessen Palladiumgehalt 0,1 bis 0,6 Gewichtsprozent beträgt, bei einem absoluten Druck von 5 bis 50 bar und einer Temperatur von 20 bis 100 °C durchführt.

2. Verfahren nach Anspruch 1, dadurch gekennzeichnet, daß man als Aluminiumoxid-Träger γ-Aluminiumoxid verwendet.

3. Verfahren nach den Ansprüchen 1 und 2, dadurch gekennzeichnet, daß der Palladium-Aluminium-Träger-Katalysator 0,3 bis 0,5 Gewichtsprozent Palladium enthält.

4. Verfahren nach den Ansprüchen 1 bis 3, dadurch gekennzeichnet, daß das Trägerkorn ein gesamtes spez. Porenvolumen von 0,45 bis 0,50 cm³/g hat.

5. Verfahren nach den Ansprüchen 1 bis 4, dadurch gekennzeichnet, daß 45 bis 55 %, vorzugsweise 50 % des Porenvolumens auf Poren entfallen, deren Durchmesser > 7,5 nm ist.

6. Verfahren nach den Ansprüchen 1 bis 5, dadurch gekennzeichnet, daß die Poren eine innere Oberfläche von 140 bis 155 m²/g, vorzugsweise 150 m²/g, besitzen.

**Claims**

1. A process for the production of n-butyraldehyde by selective hydrogenation of crotonaldehyde, characterised in that the hydrogenation is carried out in liquid phase at an absolute pressure of 5 to 50 bar and a temperature of 20 to 100 °C in the presence of a catalyst of palladium supported on aluminium oxide, the palladium being present in a peripheral zone having a thickness of 0.05 to 0.2 mm in the carrier particles, the carrier having a total specific pore volume of 0.4 to 0.6 cm³/g with from 40 to 60 % of the pore volume being in the form of pores of diameter in excess of 7.5 nm and also having a specific internal surface area of 130 to 160 m²/g and the palladium content of the catalyst being from 0.1 to 0.6 % by weight.

2. A process according to claim 1, characterised in that α-aluminium oxide is used as the aluminium oxide carrier.

3. A process according to claim 1 or 2, characterised in that the catalyst contains 0.3 to 0.5 % by weight of palladium supported on aluminium oxide.

4. A process according to any of claims 1 to 3, characterised in that the carrier particles have a total specific pore volume of 0.45 to 0.50 cm³/g.

5. A process according to any of claims 1 to 4, characterised in that 45 to 55 %, preferably 50 %, of the pore volume is in the form of pore having a diameter in excess of 7.5 nm.

6. A process according to any of claims 1 to 5, characterised in that the pores have an internal surface area of 140 to 155 m²/g, preferably 150 m²/g.

**Revendications**

1. Procédé pour la préparation continue de n-butyraldéhyde par hydrogénation sélective de crotonaldéhyde, caractérisé par le fait que l'on effectue l'hydrogénation en phase liquide à une pression absolue de 5 à 50 bar et à une température de 20 à 100 °C, en présence d'un catalyseur au palladium à support d'oxyde d'aluminium qui contient le palladium dans une zone marginale du grain de support qui a une épaisseur de 0,05 à 0,2 mm, dont le support, ayant un volume spécifique total de pores de 0,4 à 0,6 cm³/g, présente de 40 à 60 % du volume de pores sous la forme de pores ayant un diamètre supérieur à 7,5 nm avec une surface intérieure spécifique de 130 à 160 m³/g et dont la teneur en palladium est de 0,1 à 0,6 % en poids.

2. Procédé selon la revendication 1, caractérisé par le fait que, comme support d'oxyde d'aluminium, on utilise l'oxyde d'aluminium γ.

3. Procédé selon les revendications 1 et 2, caractérisé par le fait que le catalyseur au palladium à support d'oxyde d'aluminium contient de 0,3 à 0,5 % en poids de palladium.

4. Procédé selon les revendications 1 à 3, caractérisé par le fait que le grain de support a un volume spécifique total de pores de 0,45 à 0,50 cm³/g.

5. Procédé selon les revendications 1 à 4, caractérisé par le fait que 45 à 55 %, de préférence 50 % du volume de pores, sont représentés par des pores dont le diamètre est supérieur à 7,5 nm.

6. Procédé selon les revendications 1 à 5, caractérisé par le fait que les pores ont une surface intérieure de 140 à 155 m²/g, de préférence de 150 m²/g.